# EUROPEAN PATENT SPECIFICATION

(11) **EP 3 472 686 B1**
(45) Date of publication and mention of the grant of the patent: **31.07.2024**
(21) Application number: 17724024.9
(22) Date of filing: 16.05.2017
(51) Int. Cl.: G06F 3/01, G06Q 30/02

(54) **A COMMUNICATION SYSTEM**
KOMMUNIKATIONSSYSTEM
SYSTÈME DE COMMUNICATION

(30) Priority: 21.06.2016 GB 201610821
(43) Date of publication of application: 24.04.2019
(73) Proprietor: Flashy Limited, New Cross, London SE14 6NW (GB)
(72) Inventor: FREEMAN, Jonathan, New Cross London SE14 6NW (GB); ESSAFI, Daniel, Greaves Park Lancaster LA1 4TZ (GB)
(74) Representative: Beck Greener LLP
(86) International application number: PCT/EP2017/061777
(87) International publication number: WO 2017/220262

(56) References cited:
- EP-A2- 1 503 314
- WO-A1-2014/073983
- WO-A1-2015/026862
- US-A1- 2005 234 774
- US-A1- 2015 262 230

## Description

The present invention relates to a communication system.

In everyday life when a user goes about their normal business, their psychological or cognitive state affects how they interact with the world around them. It might manifest in terms of the way they operate a machine or device or the way they simply feel with respect to an item. This is true in a general sense and applies in any number of possible environments. For example, in an industrial manufacturing establishment, the cognitive state of a user affects how they physically interact with a machine they might be using. The cognitive state of a user could therefore affect how safe the user is to operate the machine. If they are angry they are likely to activate controls, e.g. push buttons or slide levers, in a quicker or more physically violent manner. In contrast, if they are happy and relaxed they might do so in a correspondingly more fluid manner. If they are tired, their actions might be slower. This variation in control inputs can change the response of the machinery. The cognitive state of a user can affect the output from the machinery under the user's control.

In a different environment, e.g. a retail environment such as a clothes shop, a user's actions can be used to determine or infer an attitude towards a particular item for sale.

EP-A-1,328,421 discloses a method of assessing vehicle operator performance. The method includes the steps of receiving vehicle operating data; monitoring an interior portion of the vehicle and receiving operator activity data from the interior portion vehicle; receiving vehicle environment data from the environment external to the vehicle; monitoring the vehicle operator and receiving operator condition data; and determining an operator assessment value. The operator assessment value is based upon the vehicle operating data, the operator activity data, the environment data and the operator condition data and is indicative of vehicle operator performance. Relevant prior art is disclosed in US 2015/0262230 A1, EP1503314 A2, WO 2015/026862 A1, US 2005/0234774 A1, WO 2014/073983 A1.

According to a first aspect of the present disclosure, there is provided, a communication system, the system comprising a receiver for receiving a signal from a transmitter associated with an item; a processor arranged to infer based on parameters of the received signal a user's psychological state with respect to the associated item.

In an embodiment, the system includes a display to display a corresponding image in dependence on the inferred psychological state.

A communication system is provided that enables a user's psychological state with respect to an associated item to be inferred. By using a transmitter associated with an item parameters relating to the item can be communicated to a processor to enable inference of a user's psychological state. The psychological state could be or could include association with the item in the first place.

The parameters could include factors such as the relative separation of the item from the user, the relative movement or acceleration of a user with respect to an item as well as environmental factors such as temperature, humidity, ambient light and the like. Typically, the system will include one or more transmitters each including some sensors for detecting movement or environmental parameters for transmission. Preferably, the transmitter is arranged to transmit Bluetooth, e.g. BLE, signals to the processor or reader.

In an embodiment, the processor receives proximity data to indicate proximity of the user to the item. The proximity sensor can detect variation in proximity of a user to the item over time. This data can be used to determine or infer a user's cognitive state.

In an embodiment, the processor receives motion data to indicate relative movement between the user and the item.

In an embodiment the receiver is an RF receiver. In one example the receiver is a Bluetooth receiver.

In an embodiment, the receiver is incorporated into a mobile telephone or personal digital assistant.

In an embodiment, the image is displayed as part of a graphical user interface on the mobile telephone screen.

In an embodiment, the system is responsive to a user interaction with the GUI to mark the item accordingly.

In an embodiment, inference of a user's psychological state with respect to the associated item is made based on whether or not the receiver is closer than 50cm to the item for more than a minimum threshold duration.

In an embodiment, the minimum threshold duration is 30s, or more preferably 20s, 10s or even 6s. The actual minimum duration can be varied in dependence on the article or type of article. For example, a determination can be made if an item has been picked up and is moving in synchrony with a user for some minimum amount of time such as 6s.

In an embodiment, the processor is arranged to determine if the movement of the user with respect to the item undergoes a defined pattern of movements followed by stationary periods and if it does to present the item to the user via the GUI. By detecting relative position or movement, a profile representing variation of position or movement of a user over time can be determined. This can be compared to some predetermined or know profiles and therefore a user's cognitive state determined accordingly.

According to a second aspect of the present disclosure, there is provided a communication system, the system comprising a receiver for receiving communications from a transmitter associated with an item; a plurality of transmitters each associated with a corresponding item; a processor arranged to infer based on parameters of the received signal a user's psychological state with respect to the associated item; and a display to display a corresponding image in dependence on the inferred psychological state.

In an embodiment the receiver is an RF receiver. In one example the receiver is a Bluetooth receiver.

In an embodiment, the transmitters are each integrated into an item associated with an article of clothing.

In an embodiment, the item is selected from the group consisting of a model, a coat hanger, a mannequin or a supporting shelf.

According to a second aspect of the present disclosure, there is provided a communication system, the system comprising a receiver for control and management by a user, the receiver, in use, being arranged to receive a signal from a transmitter associated with an item; a processor arranged to infer based on parameters of the received signal a user's psychological state with respect to the associated item.

In an embodiment, the communication system comprises plural transmitters, each associated with a corresponding to an item and being arranged to transmit signals for receipt by the receiver.

In an embodiment, the processor is arranged to determine a variation in received signal strength over time and to compare the determined variation with stored profiles to identify a match and thereby to infer a user's cognitive state with respect to the item.

In an embodiment, each transmitter is associated with or coupled to a particular coat hanger. In an embodiment, in addition or in the alternative, one or more transmitters may be associated with an in-store display and/or a display rail and/or a window display viewed from outside the retail environment/shop.

According to a third aspect of the present disclosure, there is provided a communication method, the method comprising receiving a signal from a transmitter associated with an item; inferring based on parameters of the received signal a user's psychological or cognitive state with respect to the associated item.

In an embodiment, the method comprises displaying a corresponding image in dependence on the inferred psychological state. The image preferably includes an image of the item or an indicator or description thereof.

The invention is defined in independent claim 1. Embodiments will now be described in detail with reference to the accompanying drawings, in which:
Figures 1 to 3 are schematic representations of communication systems;
Figure 4 is a schematic representation of an example of a series of graphical user interfaces for use with the communication system of Figure 3;
Figure 5 is a flow diagram showing an example of a basic algorithm that could be sued in the example of the communication system of Figures 3 and 4;
Figure 6 shows an example of a typical variation is signal strength as detected at a receiver in a communication system; and
Figure 7 is a schematic representation of a communication system.
Figure 8 is a schematic representation of a communication system; and
Figure 9 is a schematic representation of a flow diagram for understanding operation of the communication system of Figure 8.

In an embodiment, the present communication system includes a receiver for receiving communication from a transmitter associated with an item. Based on the parameters of the received signal, a user's psychological or cognitive state with respect to the associated item can be inferred or determined and optionally, a corresponding image can be displayed to a user based on the inferred psychological state. The psychological state could, for example, include any aspect of cognitive state that might be relevant to the user's interaction or engagement with the item in question. It could indicate any one or more of a general interest in, a liking or appreciation of or a desire for the item in question.

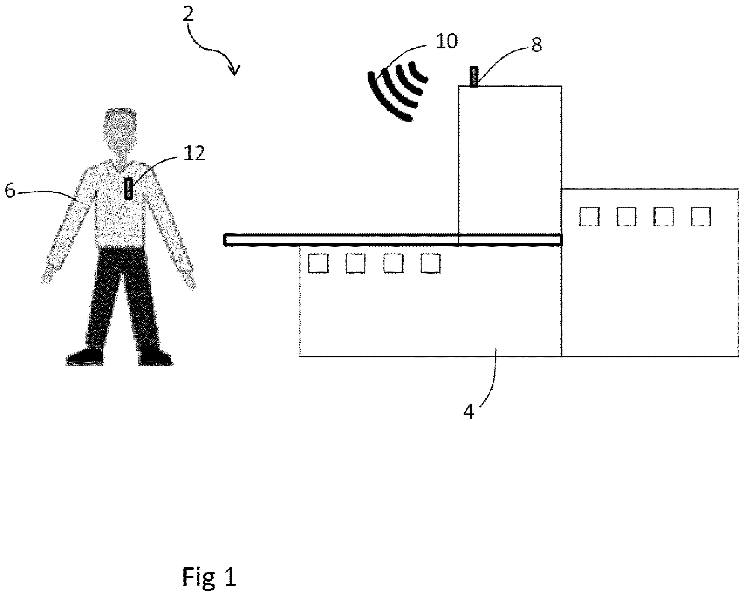

Referring first to Figure 1, an example of a communication system 2 is shown for use with an industrial manufacturing system. The system 2 comprises a manufacturing apparatus 4. A user or operator 6 in normal operation will interact with the apparatus 4 to carry out its usual process and functions. A transmitter 8 is provided as part of a communication system and is arranged to transmit a signal such as an RF signal 10 to an area close to the apparatus 4 within which the operator 6 will, in use, typically be positioned. The operator 6 has a receiver 12 for receiving communications from the transmitter 8. The relative time during which an operator 6 is positioned in proximity to the apparatus 4 or his relative movement to the apparatus 4 can be detected and inferred by the receiver 12 by measurement of the received signal 10 from the transmitter 8. In other words, if a user simply remains stationary for an extended period of time such that a constant, non-varying signal is received by the receiver 12 over an extended period time, then it can be inferred or deduced that the user has been operating the apparatus continuously without break for the corresponding length of time.

If the period of time for which the user has been in proximity to the apparatus for exceeds some defined threshold, then it can be inferred that the operator may be tired or need a break from operating the apparatus 4. A display typically, although not necessarily, associated with the receiver 12, e.g. provided as part of a user's mobile telephone or PDA (not shown), can be activated or controlled so as to generate a warning or some form of indicative signal to the operator 6.

In other words by receiving at the receiver 12 the communications from the transmitter 8 a process within the receiver is able to infer the user's psychological state with respect to the apparatus 4.

As well as simply operating based on the amount of time that a user 6 has been close to the apparatus 4, the detector 12 under control of the operator 6 may be able to determine relative movement of the user 12 with respect to the apparatus. This could be achieved, for example, by detecting variation with respect to time in the strength of the transmitted signal 10 from the transmitter 8. The transmitter 8 is preferably configured to transmit a continuing constant power signal 10 and therefore variation of the received strength of the signal at the receiver 12 will be due to changes in relative position between the user 6 and the apparatus 4. In other words, it will be due to movement of the operator 6 with respect to the apparatus 4. Given that in certain environments, a state of agitation of an operator might indicate a lower degree of concentration on the task at hand, it could be that a controller associated with the apparatus 4, when it is detected that the operator is moving more than some defined threshold, is arranged or configured to introduce a further checking step within the control signals for operation of the apparatus 4. In other words, the processor is arranged to infer based on parameters of the received signal, such as duration or variation in strength, a user's psychological state with respect to the associated item. Corresponding action can then be taken.

In the example described above the use of a transmitter associated with the item and a receiver associated with user is described. In another example, instead of a transmitter associated with the item some form of data gathering device or monitor that does not include the transmission of a signal could be used. For example, an imaging device could be associated with the item and could be arranged to monitor the position or movement of the user with respect to the item or apparatus 4.

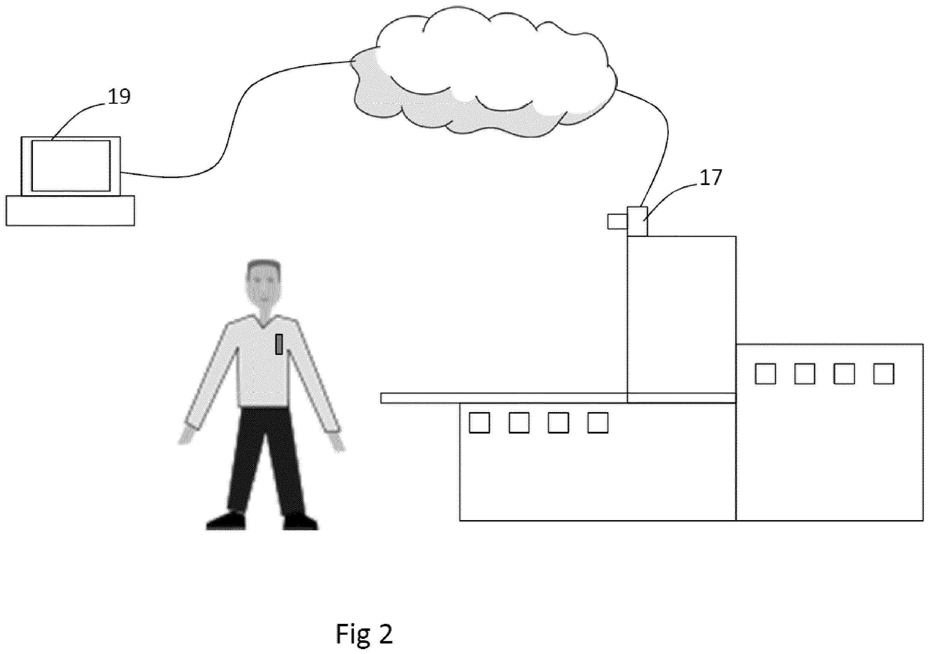

Figure 2 shows a further example of operation of the communication system. In this case, apparatus 4 is shown schematically and may be an industrial system for production of, say, extruded PVC beams. An operator is shown in proximity to the apparatus. A camera 17 is provided arranged such that it captures objects and activity within an operating zone in relatively close proximity to the apparatus 4. The camera is connected via hard wired connection or wirelessly to a server 19. The server 19 is shown in this example as being cloud-based. The server 19 has running on it software arranged to process the received image data and to infer from it the cognitive or psychological state of the operator. As described above this may be determined based on algorithms which determine the relative proximity, dwell time, movement and the like of the user with respect to the apparatus 4. In this example since a camera is used, images of the user's face and/or posture could be communicated to the server and then facial recognition software utilised to infer the cognitive state of the user.

Responsive to the determined cognitive state the operation of the apparatus 4 can be controlled automatically so as to allow for expected performance variation of the user. For example, if the movement of the operator is determined to be slow or slower than usual then it can be inferred that he might be tired and so an additional level of confirmation may be requested by the operating system of the apparatus when receiving a control input. The user may have a receiver with him which is configured to receive a notification from the server 19 in the event of any change or particular status as regards cognitive state. In other words, independently of any direct sensory information determined by the user's physical interaction with the apparatus itself, the system is able to infer the cognitive or psychological state of the user and take action accordingly.

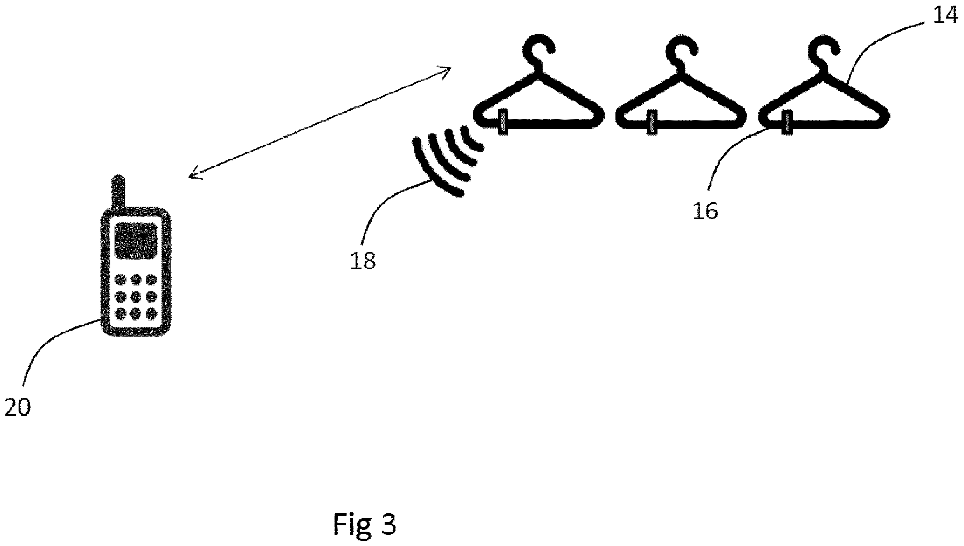

In both the examples described above, the user is clearly associated with a particular piece of machinery or equipment. Figure 3 shows a further example of operation of the communication system, where as well as or instead of, the system is used to match a user with an item. The matching of a user to an item can be thought of as the psychological state of the user with respect to the item although further aspects of psychological state can be identified, as explained in detail below. In this example, the communication system is deployed within the environment of a retail outlet such as a clothes shop. In this context, the inventors refer to the system as the Emotionally Intelligent Retail Engine (EIRE), where based on a user's psychological state, images, offers and communications appropriate to that state can be served to the user by the EIRE.

A plurality of clothes hangers 14 are provided each having associated with it a transmitter 16. The transmitters 16 are arranged to transmit a signal 18 which, in use, will be detected by a receiver within a user's mobile telephone 20 (or other device such as a tablet, portable computer, iPod, Wi-Fi device, smart device, smart watch etc). In this example, by detecting the dwell time or period within which a user is located close to the transmitter 16, it is possible to infer an interest in the user 6 with the item associated with the hanger 14 or transmitter 16 whose signal has been received.

Thus, it will be appreciated, that not only can the correlation, motion and proximity data be used to learn more about the mental state of the user with respect to an item, but by definition it can also been used to associate the user with the item, i.e. to identify which user is with which item. This is a significant aspect of the present system since whereas in many known systems it is known that data from a particular sensor is related to a particular person because, say, he is wearing it or there is some other clear and definite link between sensor and person, this is not the case with the present system in a retail environment. Indeed, in the present system when used in the retail environment, there may be a plurality of people with access to and ability to handle a plurality of items in the specific environment. The system serves to associate items with people, and can then use the sensor data to work out further psychological states of a user with respect to the identified or user-matched item.

In a preferred example, the transmitters 16 are Bluetooth transmitters and are arranged to communicate with a Bluetooth receiver on a user's mobile telephone. With this combination of elements, it is possible to infer based on behaviour of the user (determined using the received signal), a user's psychological or cognitive state with respect to the associated item. The item could be the clothing which is on the hanger 14. By detecting a variation in signal or a consistently strong signal for some defined threshold of time, it is possible to infer that a user has an interest in potentially trying on and/or purchasing the item of clothing associated with the transmitter 16.

In a preferred example, the user has downloaded onto their mobile telephone (or other such device as indicated above) an App which is configured to receive Bluetooth signals from the transmitters on the hangers and determine based on the user's activity, the cognitive state of the user with respect to the clothing on the hanger. For example, if a user stands close to a hanger in a clothes shop for a period of between 15 and 90seconds (more preferably between 15 and 60 seconds), then based on a defined algorithm, the App will determine that the user's cognitive state with respect to the clothing item is positive. A display on the user's telephone then offers the user the option of placing the item within a clothing wishlist.

By interacting with the display a user is able to populate a wish list of items that he likes. In other words, based on a user's behaviour, the psychological state with respect to a particular item is inferred and then an invitation may be sent to the user based on the inferred psychological state which enables the user to easily and simply include the item in a wish list.

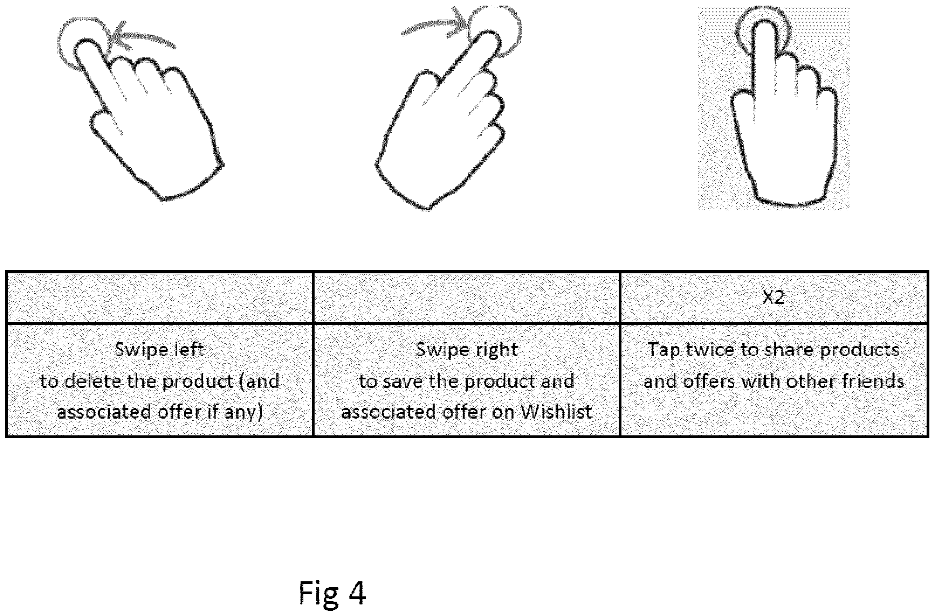

Referring to Figure 4, the process of user interaction with a graphical user interface when in a retail environment can be seen. The situation that has arisen is that a user has visited a shop, such as a clothes shop, the user has dwelled for a defined period of time by a display or by an item of clothing associated with a transmitter on a hanger or a shelf. Communication between the transmitter and the user mobile telephone has ensued, as described above. A notification or alert is sent to the user, such as vibration of the handset or an audible notification. This indicates to the user that communication has taken place between a transmitter and his mobile telephone regarding a particular item. Figure 4 shows the possible responses that a user can take to the notification. A first option would be to swipe left which would simply delete the product and any associated offer. The second option would be to swipe right which would save the product and associated offer in a wish list. A third option would be to tap the notification twice to share the products and offers with other friends, such as via a social network.

Users are notified of real-time offers on products that are on their wish list. The user will be reminded when a product has been added to their wish list goes on sale. However, a user is provided with complete control over the reminders and notifications that they receive and is able to switch these off if desired. A user is able to decide on which types of, when and how many notifications they want to receive and they can also set the number of days that must pass before notifications are removed. A dedicated app can be provided on the user's mobile telephone to enable all of this functionality to be achieved. Furthermore the App could enable interaction with delivery service or online shopping such that from the interface offering inclusion of the item on the wish list a user can directly chose to buy the article or item in question.

Referring again to the example of Figure 1, a display screen or monitor could be provided as part of a user's PDA or mobile telephone or as an integrated part of the apparatus for. First, based on detection of the relative position and of movement of the user 6 with respect to the apparatus 4, the user's cognitive state is inferred and a display is then arranged to show an appropriate graphical user interface to the user for engagement. In this example, the GUI could indicate a question seeking confirmation. For example, it could indicate a message saying, "*You have been working for two hours without break. Need* a *rest?"*

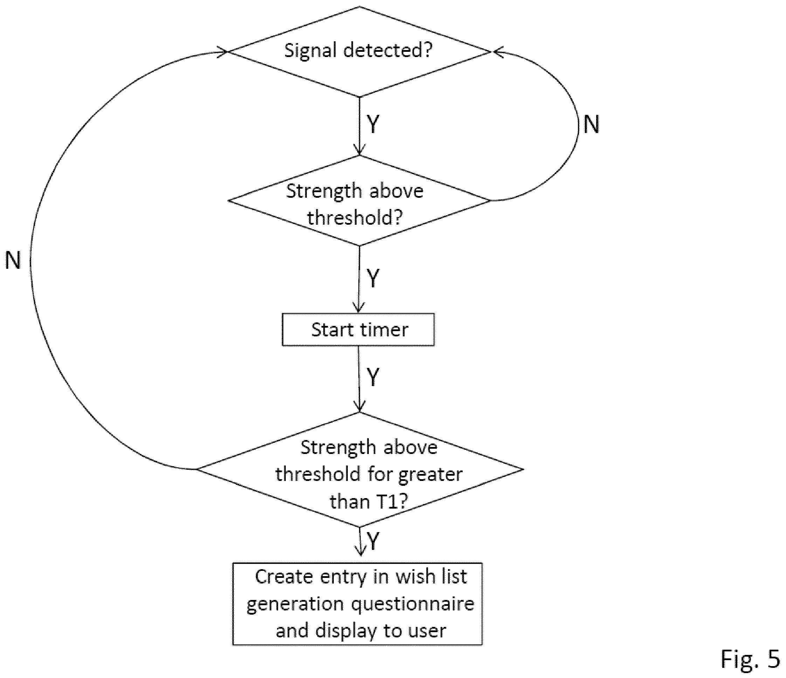

Figure 5 is a flow diagram showing an example of a basic algorithm that could be used in the example of the communication system of Figures 3 and 4. Initially a determination is made as to whether a signal such as a Bluetooth transmission is received. If it is, then the strength of the signal is determined and in dependence on this a timer is started. If the strength is greater than some defined threshold then it is assumed that the user is within some defined proximity to the transmitter and therefore is showing an interest in the item associated with the transmitter. The timer starts so as to be able to measure the length of time that the user remains within the proximity. Periodic checks are made that the strength of the signal is maintained at or above the defined threshold. If the signal strength decreases then this could indicate that the user has walked further away from the transmitter and item in question. If it is determined that for a defined period of time the signal was maintained above a threshold level, i.e. the user for the defined period of time was within the required threshold proximity to the item then the user's cognitive state with respect to the item is inferred. In other words it is determined that the user has interest in the item.

A display is generated on the user's mobile telephone such as that shown in and described above with reference to 4. In some examples a display is not provided as part of the communication system. Some other means of providing feedback to user is provided. For example instead of displaying something to a user via the screen of the mobile telephone a user response can be prompted by providing feedback in the form of a haptic response or an audible alarm being generated to get the attention or a reaction from the user.

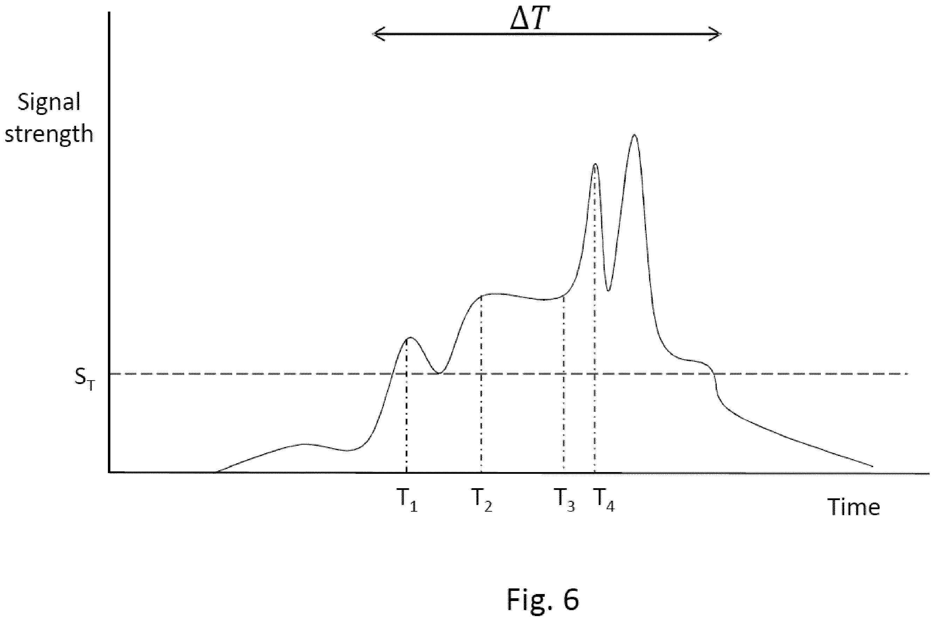

The variation of signal strength can be mapped to what would be expected for certain expected behaviour patterns of a user with respect to an item of clothing. Figure 6 shows an example of a typical variation is signal strength as detected at a receiver. In this example the situation shown is a user's interaction with an item of clothing, say a shirt, on a hanger in a clothes shop. Initially a user walks around the shop and as he does so his mobile telephone will pick up and lose the Bluetooth transmissions from plural transmitters placed around the shop as he moves closer to or further away from various items. At some point, he slows down close to a shirt and then approaches it. The strength of the detected signal therefore increases as shown by the first peak at T₁. Shortly before this a timer has started since the signal has exceeded the defined strength threshold Sr. As the user stays generally in the same area but shuffles slightly towards and away from the shirt as he looks at it there is some slight variation in signal strength. At T₂, he stands still until T₃ simply looking at the shirt. Then he picks up the shirt and brings it close to himself whilst looking in a nearby mirror. He moves the shirt away and then repeats this again bringing it even closer. This is demonstrated by the two close peaks (first one at T₄). Finally the user puts the shirt back on the clothes rack and walks away. The signal strength peters out. For a duration of ΔT the signal strength has been above the defined threshold S_{T}.

The variation in signal strength is analogous to a signal spectrum such that certain characteristics can be modelled to specific interactions. In other words, the variation in signal strength shown in the graph of figure 6 can be considered a recognisable "finger print" of a certain type of interaction. The App interprets the measured signal variation as demonstrating the user's (positive) cognitive state with respect to the item (the shirt in this example) and so can then chose to offer the option of including the shirt on a wish list, as explained above with reference to Figure 4.

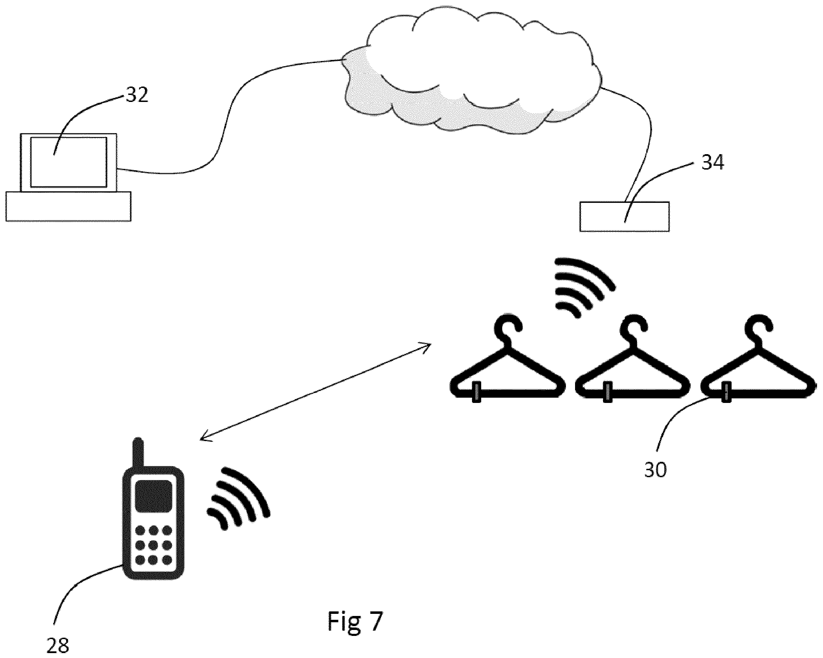

Figure 7 is a schematic representation of a communication system. The system 26 is similar to that shown in and described with reference to Figure 3. In this case however, each of the coat hangers has associated with it a transceiver arranged both to transmit and receive signals. A user's mobile telephone 28 is arranged via a downloaded App to transmit communications to the transceivers 30 associated with the coat hangers and therefore with the clothes items upon them. The transceivers are arranged to detect the signal strength and to transmit this information including the strength variation with time to a server 32. An intermediate transponder 34 may be provided and the server could be, as in the example shown, a network connected server. In this way processing of the received signal to determine a user's cognitive state can be done at the server instead as well as at the user's device 28.

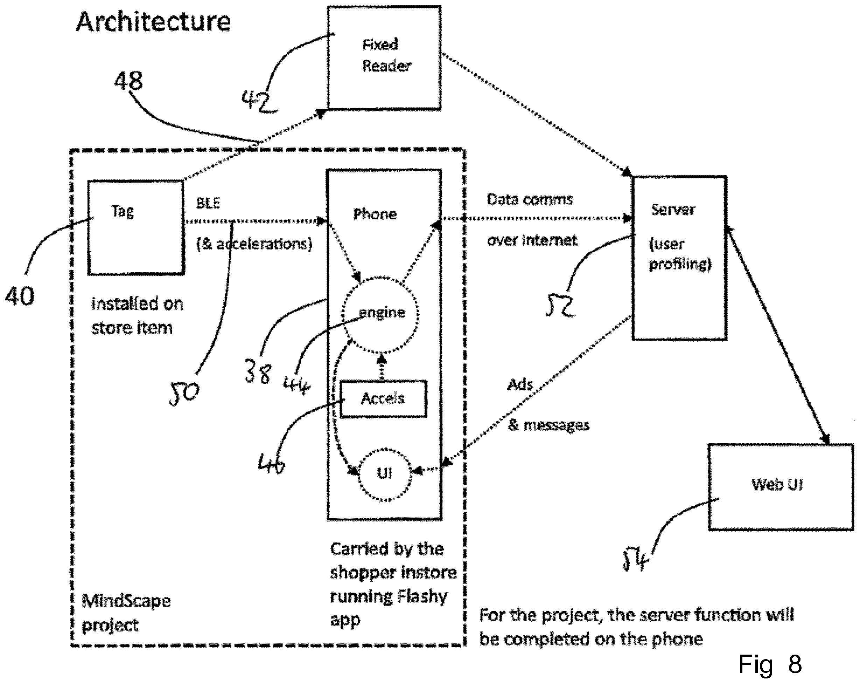

Figure 8 is a schematic representation of a communication system. The system is similar to that described with reference to Figures 1 to 3 and 7 but includes some further functionality and components.

In the example shown, a user's phone 38 is provided. One or more tags 40 are provided which will, in use, be associated with an item of clothing or a hanger in a clothes shop. In addition to the communication system of, say, Figure 3, in this example, a reader 42 is provided. The reader 42 may be fixed in that it is positioned at any point at some defined and fixed location within the environment in which the communication system is operating.

In this example, as well as the phone 38 being arranged to receive signals from the tags 40, the fixed reader 42 is also configured and arranged to do so. The fixed reader 42 is arranged to receive communications, e.g. signal transmissions from the tag 40. The transmissions from any particular tag contain data relating to parameters associated with that tag such as any or all of position, movement, acceleration and environmental data such as temperature, humidity and the like. The tag 40 preferably includes an accelerometer (not shown) for use in determining information about the tag such as the movement or acceleration it is undergoing. The tag preferably includes one or more sensors selected from the group including: an accelerometer, a thermometer, humidity sensor, light sensors, sound sensor and one or more other environmental sensors.

The user's phone 38 includes a processor or engine 44 arranged to receive data from an accelerometer 46 that is typically fitted as a standard component within a smart phone. As will be explained below, the accelerometer also included within the tag 40 moves with the tag and therefore data associated with movement and acceleration of the tag is able to be communicated both to the fixed reader 42 via a transmission 48 and with the phone 38 via a transmission 50.

The accelerometers that are preferably provided within both the smart phone 38 and the tag 40 are preferably microelectromechanical systems (MEMS) three axis accelerometers able to detect movement and acceleration in three orthogonal axes. In use, an accelerometer provided as part of tag 40 detects movement and acceleration of the tag and is arranged to encode this within payload data for transmission to the fixed reader 48 and the phone 38 via transmissions 48 and 50, respectively. The encoded data provided by the tag will typically include some or all of signal time of flight, signal angle of arrival, location data, angular rotational movement (which may be derived from one or more gyroscopes provided within the tag) and movement and acceleration in three dimensions.

Preferably, the transmitter within the tag 40 is a Bluetooth low energy (BLE) signal transmitter and in use is attached to a hanger. When a BLE signal is transmitted by the tag it is received at the phone 38 and/or fixed reader 42 where the strength is measured and the signal can be decoded. In other words, preferably the fixed reader 42 is provided with some processing capacity to enable it to analyse the data and thereby to infer based on the received signal a user's psychological state with respect to the item associated with the tag 40. The fixed reader is so called since in practice it will be permanently or semi-permanently fixed at a location that enables it to receive signals from as many tags 40 as possible. In some examples though it might only be "fixed" in the short term in the way that, say, a landline telephone base with a portable handset is fixed. In other words it can easily be picked up and moved somewhere else but in normal use as compared to the tags from which it is receiving data it is a "fixed base".

The tag thus includes various sensors which serve to measure parameters associated with the tag and then transmit the data to a receiver in the telephone in BLE data packets. The acceleration of the phone 38 in three dimensions are measured by the internal accelerometer/sensor within the phone. Most smart phones nowadays include such a component or functionality. These may be provided to a server 52 which will be explained in greater detail below. In addition the data received by the fixed reader 42 from a tag 40 is also communicated to the server 52. In the example shown, data is communicated to the server 52 from the phone 38 over an internet connection. In an alternative embodiment, BLE communication between the phone and the fixed reader 42 might be enabled in which case the data from the phone itself will also be provided to the server 52 from the fixed reader 42 directly and not from the phone itself.

Variations in the measured parameters with time are detected and transmitted to the fixed reader and/or server 52. Thus a plurality of time series are measured and provided to the server and/or the fixed reader 42. By receiving the various time series from different sensors on tags and also the phone, the server 52 is able to perform analysis by which it is possible to infer the state of mind of the user of the phone 38. The server 52 might for example be programmed and arranged to perform a correlation of a time series received from a tag 40 and from a phone 38 and determine from the correlation that the owner of the phone was of a certain mind-set with respect to the item associated with the tag 40.

In the example given above with respect to Figure 6 it was explained above how based on relative separation of a user and a tag it is possible to infer something about the user's state of mind. In the present example the same inference can be drawn and also a similar assessment of a user's frame of mind with respect to an item can be made except instead of relying predominantly (or only) on distance between or separation of a user and an article, other parameters are taken into account.

As explained above, communications can then be sent from the server 52 to a user interface on the phone 38 for communication with the user. Thus, as explained above, if it is determined based on a correlation between accelerometer data received in respect of a tag 40 and accelerometer data received in respect of a telephone 38, that a user has an interest in a particular item associated with the tag 40, an offer can be sent from the server 52 to the user which the user can then choose to either accept or delete/ignore.

A web user interface 54 is provided which enables management and control of and action in dependence on the received data from the fixed reader 42 and the phone 38. The web user interface 54 can be arranged to provide various levels of interaction with the received and processed data. For example, on one level it might simply be used for direct management within a retail environment such as by a store manager who may receive via the interface a notification that a particular user is interested in a particular item. This could prompt the store manager to approach the user and offer something with respect to the article, such as a discount or merely to engage in conversation with the user in the knowledge that there is a higher chance that the user might actually want to purchase the article in question.

On a further or higher level of management, it is possible that data could be aggregated from the server in respect of a plurality of readers and/or mobile telephone which would enable an understanding of overall behaviour patterns within a particular environment to be understood.

In one example, even when a user does not have a telephone which is arranged to interact with the tags directly, the user simply choosing to interact with the tags 40 will prompt data to be transmitted to the fixed reader 42. The server 52 on receiving data from a plurality of tags 40 will be able to generate a useful level of understanding regarding the tags and items associated with the tags. For example, over an extended period of time, such as say, one month, it might be noticed that when a particular clothes rack containing hangers with a certain line of clothing is positioned in one area of a store, an increased level of user interaction with the tags is prompted. This is useful data irrespective of any information regarding whether a particular single user has an interest in purchasing the article in question. What it enables a store or business manager to do is understand that positioning certain types of clothing within certain areas within a shop has a beneficial effect in terms of sales of that article of clothing. Again, as above this can be achieved by determining time series of data received from tags. By recognising patterns within the time series a useful body of data can be built up.

In addition, where data other than simply movement or acceleration of the tags is used the same principles apply. For example if temperature data is measured by one or more tags and transmitted as part of a BLE signal to the reader 42 then a management application interacting with the user interface 54 can infer that, say, if the temperature within a store is varied in accordance with some timetable or schedule, then sales can be maximised. In other words, the server 52 can act as a data gatherer by receiving data from tags or readers 42 (which themselves are receiving data from tags 40). Thus, even without direct interaction with a user device 38, useful information can be derived.

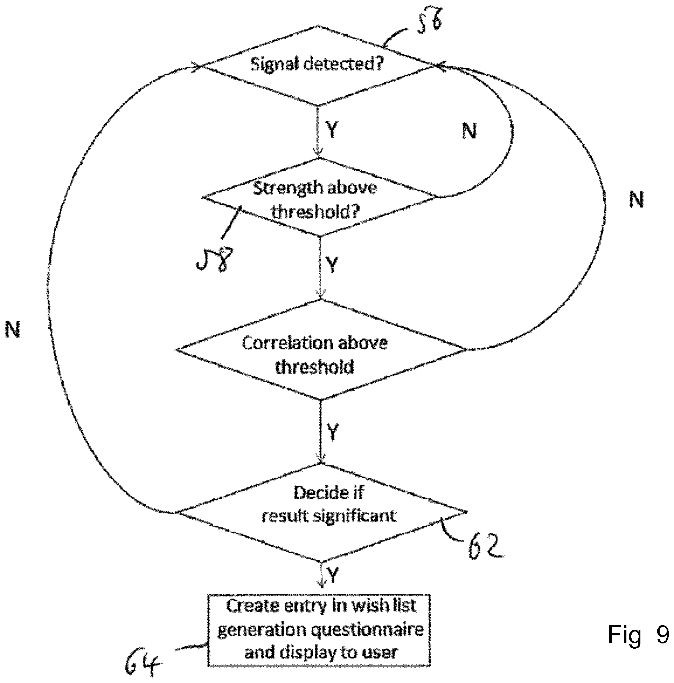

Figure 9 is a schematic representation of a flow diagram for further understanding operation of the communication system of Figure 8.

In the example shown, initially, at step 56 a determination is made as to whether or not a signal is detected. If it is, the determination is made, at fixed reader 42 as to whether or not the strength of the signal is above a certain threshold.

The signal detection 56 might include data both from a tag 40 and a user mobile telephone 38. At the server 52, a determination is made as to whether or not there is sufficient correlation between the data received from the tag 40 and that received from the phone 38. If there is, a further determination at step 62 is made as to whether or not the result is significant, i.e. whether or not any offer or interaction with a user is required based on the determined result. If it is, then at step 64, an entry is generated in a wish list for display to a user.

Embodiments have been described with particular reference to the examples illustrated. However, it will be appreciated that variations and modifications may be made to the examples described within the scope of the present invention, which is defined by the claims.

## Claims

1. A communication system, the system comprising
- a tag (40) associated with an item, wherein the tag includes an accelerometer and a transmitter;
- a fixed reader (42) for receiving a signal from said transmitter, said signal comprising acceleration data from said accelerometer;
- a mobile telephone (38) also comprising an accelerometer (46);
- a server (52) being arranged to receive respective acceleration data from the mobile phone and from the fixed reader, and to infer a user's psychological state with respect to the item associated with the tag (40) based on correlation between accelerometer data received in respect of the tag (40) and accelerometer data received in respect of the mobile telephone (38).

2. A communication system according to claim 1, comprising a display to display a corresponding image of the item or an indicator or description thereof in dependence on the inferred psychological state.

3. A system according to any of claim 1 to 2, in which the tag 40 is a Bluetooth transmitter, in particular a Bluetooth low energy (BLE) signal transmitter.

4. A system according to any of claims 1 to 3, in which the system comprises a display to display a corresponding image in dependence on the **inferred psychological** state and the image is displayed as part of a graphical user interface on the mobile telephone screen.

5. A system according to any of claims 1 to 4, wherein the tag includes furthermore one or more sensors selected from the group including: a thermometer, humidity sensor, light sensors, sound sensor and one or more other environmental sensors.

6. A communication system according to any of claims 1 to 4, the system further comprising:
a plurality of tags (40), each having a corresponding transmitter,
each tag also including an accelerometer;
and
a display to display a corresponding image in dependence on the inferred psychological state.

7. A system according to claim 6, in which the tags transmitters are each integrated into an item associated with an article of clothing, in which the item is selected from the group consisting of a model, a coat hanger, a mannequin or a supporting shelf.

8. A communication system according to claim 7, each associated with a corresponding to an item and being arranged to transmit signals for receipt by the receiver.

## Patentansprüche

1. Ein Kommunikationssystem, wobei das System umfasst
∘ ein mit einem Gegenstand verbundenes Etikett (40), wobei das Etikett einen Beschleunigungsmesser und einen Sender umfasst,
∘ ein ortsfestes Lesegerät (42) zum Empfangen eines Signals von dem Sender, wobei das Signal Beschleunigungsdaten von dem Beschleunigungsmesser umfasst;
∘ ein Mobiltelefon (38), das außerdem einen Beschleunigungsmesser (46 ) umfasst;
∘ einen Server (52), der dazu eingerichtet ist, jeweilige Beschleunigungsdaten vom Mobiltelefon und vom festen Lesegerät zu empfangen und auf der Grundlage einer Korrelation zwischen bezüglich des Etiketts (40) empfangenen Beschleunigungsmesserdaten und bezüglich des Mobiltelefons (38) empfangenen Beschleunigungsmesserdaten auf den psychologischen Zustand eines Benutzers bezüglich des mit dem Etikett (40) verknüpften Gegenstands zu schließen.

2. Ein Kommunikationssystem nach Anspruch 1, das eine Anzeige zum Anzeigen eines entsprechenden Bildes des Gegenstands oder eines Indikators oder einer Beschreibung davon in Abhängigkeit von dem abgeleiteten psychologischen Zustand umfasst.

3. Ein System gemäß einem der Ansprüche 1 bis 2, bei dem das Etikett (40) ein Bluetooth-Sender, insbesondere ein Bluetooth Low Energy (BLE)-Signalsender ist.

4. Ein System gemäß einem der Ansprüche 1 bis 3, bei dem das System eine Anzeige zum Anzeigen eines entsprechenden Bildes in Abhängigkeit von dem abgeleiteten psychologischen Zustand umfasst und das Bild als Teil einer grafischen Benutzeroberfläche auf dem Bildschirm des Mobiltelefons angezeigt wird.

5. Ein System gemäß einem der Ansprüche 1 bis 4, wobei das Etikett außerdem einen oder mehrere Sensoren umfasst, ausgewählt aus der Gruppe, die ein Thermometer, einen Feuchtigkeitssensor, Lichtsensoren, einen Geräuschsensor und einen oder mehrere andere Umweltsensoren umfasst.

6. Ein Kommunikationssystem nach einem der Ansprüche 1 bis 4, wobei das System weiterhin umfasst:
∘ eine Vielzahl von Tags (40), von denen jedes über einen entsprechenden Sender verfügt, wobei jedes Tag außerdem einen Beschleunigungsmesser umfasst;
∘ und eine Anzeige zum Anzeigen eines entsprechenden Bildes in Abhängigkeit von dem abgeleiteten psychologischen Zustand.

7. • Ein System gemäß Anspruch 6, bei dem die Tag-Sender jeweils in ein mit einem Kleidungsstück verbundenes Objekt integriert sind, wobei das Objekt aus der Gruppe ausgewählt ist, die aus einem Modell, einem Kleiderbügel, einer Schaufensterpuppe oder einem Trägerregal besteht.

8. • Ein Kommunikationssystem gemäß Anspruch 7, das jeweils einem entsprechenden Gegenstand zugeordnet ist und zum Übertragen von Signalen zum Empfang durch den Empfänger eingerichtet ist.

## Revendications

1. Système de communication, le système comprenant
∘ une étiquette (40) associée à un article, l'étiquette comprenant un accéléromètre et un émetteur ;
∘ un lecteur fixe (42) pour recevoir un signal dudit émetteur, ledit signal comprenant des données d'accélération dudit accéléromètre ;
∘ un téléphone mobile (38) comprenant également un accéléromètre (46 ) ;
∘ un serveur (52) étant agencé pour recevoir des données d'accélération respectives du téléphone mobile et du lecteur fixe, et pour déduire l'état psychologique d'un utilisateur par rapport à l'élément associé à l'étiquette (40) sur la base d'une corrélation entre les données d'accéléromètre reçues en ce qui concerne de l'étiquette (40) et des données d'accéléromètre reçues concernant le téléphone mobile (38).

2. Système de communication selon la revendication 1, comprenant un affichage pour afficher une image correspondante de l'élément ou un indicateur ou une description de celui-ci en fonction de l'état psychologique déduit.

3. Système selon l'une quelconque des revendications 1 à 2, dans lequel l'étiquette (40) est un émetteur Bluetooth, en particulier un émetteur de signal Bluetooth Low Energy (BLE).

4. Système selon l'une quelconque des revendications 1 à 3, dans lequel le système comprend un affichage pour afficher une image correspondante en fonction de l'état psychologique déduit et l'image est affichée en tant que partie d'une interface utilisateur graphique sur l'écran du téléphone mobile.

5. Système selon l'une quelconque des revendications 1 à 4, dans lequel l'étiquette comprend en outre un ou plusieurs capteurs sélectionnés dans le groupe comprenant : un thermomètre, un capteur d'humidité, des capteurs de lumière, un capteur sonore et un ou plusieurs autres capteurs environnementaux.

6. Système de communication selon l'une quelconque des revendications 1 à 4, le système comprenant en outre :
∘ une pluralité d'étiquettes (40), chacune ayant un émetteur correspondant, chaque étiquette comprenant également un accéléromètre ;
∘ et un affichage pour afficher une image correspondante en fonction de l'état psychologique déduit.

7. • Système selon la revendication 6, dans lequel les émetteurs d'étiquettes sont chacun intégrés dans un article associé à un article vestimentaire, dans lequel l'article est choisi dans le groupe constitué d'un mannequin, d'un cintre, d'un mannequin ou d'une étagère de support..

8. • Système de communication selon la revendication 7, chacun étant associé à un élément correspondant à un élément et étant agencé pour transmettre des signaux destinés à être reçus par le récepteur.
